# EUROPEAN PATENT SPECIFICATION

(11) **EP 1 421 962 B1**
(45) Date of publication and mention of the grant of the patent: **10.12.2008**
(21) Application number: 02026375.2
(22) Date of filing: 25.11.2002
(51) Int. Cl.: A61M 5/32

(54) **Disposable syringe**
Einwegspritze
Seringue jetable

(43) Date of publication of application: 26.05.2004
(73) Proprietor: Shue, Ming-Jeng, Hsi Dist, Taichung City (TW); Huang, Deborah, Taichung City (TW)
(72) Inventor: Shue, Ming-Jeng, Hsi Dist, Taichung City (TW); Huang, Deborah, Taichung City (TW)
(74) Representative: Serjeants

(56) References cited:
- FR-A- 2 675 999
- US-A- 5 084 018
- US-A- 5 114 410
- US-A- 5 176 640
- US-A- 5 211 629
- US-A- 5 385 551

## Description

This invention relates to a disposable syringe, more particularly to a disposable syringe which enables a needle cannula to be retracted within a plunger cavity having a reduced pressure therein.

Referring to Figs. 1 and 2, U.S. patent No. 5,176,640 discloses a hypodermic injection syringe 10 which includes a barrel 11, a base 12 fixed to an open front end 111 of the barrel 11, a needle cannula 13 having a rear needle end 132 that extends into the barrel 11 through the base 12 along an axis and that has an engagement recess 131, and a plunger 18 slidable within the barrel 11. The plunger 18 includes a hollow shaft 14 with a vacuum chamber 141, and a head which has an elastic rubber ring 16 with a channel that is closed by a disc 161, and a plug 15 with a tube forcibly inserted into the channel of the ring 16 and secured to the shaft 14. A piston 17 is slidable in a sealed manner within the chamber 141, and is provided with an engaging rod 172 formed with an elastic flap 171 on a front end thereof.

In use, when the plunger 18 is about to finish its injection stroke, the disc 161 is lacerated by the rear needle end 132, the rod 172 then engages the needle 13 by engagement of the flap 171 and the engagement recess 131. In this state, under the action of vacuum present in the chamber 141, the rod 172 and the needle 13 are sucked into the chamber 141.

Since the needle 13 can be retained in the chamber 141 of the plunger 18 for safety disposal, the following drawbacks arise:
1. The laceration of the disc 161 may occur when the injection procedure of the syringe 10 is not finished. Thus, some medicine or blood may will remain within the barrel 11 to result in contamination.
2. In order to smoothly suck the needle 13 into the chamber 141, the securing of the needle 13 to the base 12 cannot be very tight. However, insufficient securing of the needle 13 will result in movement or removal of the needle 13 during the injection procedure.

French Patent 2675999 discloses a syringe with two different diameters. A needle cannula is mounted on a needle seat which is itself mounted on a second needle seat 6. The second needle seat 6 has a cavity 15. A plunger with a reduced pressure cavity is used.

A hollow member 11 is held in place at the end of the plunger's cavity by means of movable radial catches 18, which form a releasable seal.

A grapple member is slidably held within the hollow member. A grapple 12 of the grapple member protrudes from the end of the plunger. On further depression of the plunger the grapple 12 locks into the cavity 15. This simply locks the parts together and there is no automatic withdrawal of the needle. Only when the plunger is pulled back a small distance, so that the grapple slides within the hollow member 11 are the catches released.

The grapple, needles seats and needle cannula are then forced back into the cavity by atmospheric pressure.

This syringe suffers from a complicated withdrawal mechanism that is not fool-proof.

The object of the present invention' is to provide a disposable syringe which can prevent trapping of medicine or blood therein after injection and which can be operated easily and smoothly to retract a used needle within a plunger.

According to this invention, the disposable syringe includes a needle cannula, a barrel, a tubular barrier member, a tubular needle seat, and a plunger. The barrel has an inner surrounding barrel wall surface surrounding an axis and confining a passage with opposite rearward and forward openings. The inner surrounding barrel wall surface includes a larger-diameter segment and a smaller-diameter segment which confine rear and front passageways, respectively, to form a surrounding shoulder portion therebetween. The smaller-diameter segment includes a smaller front surrounding region and a larger rear surrounding region to form a surrounding abutment wall therebetween. The larger-diameter segment includes proximate and distal surrounding regions respectively proximate to and distal from the surrounding shoulder portion. The proximate surrounding region has a retaining area spaced apart from the surrounding shoulder portion.

The tubular barrier member includes front and rear surrounding edge portions, and inner and outer barrier wall surfaces. The outer barrier wall surface is retained at the retaining area by virtue of a first frictional force generated therebetween while in water-tight engagement with the proximate surrounding region, thereby partitioning the rear passageway into a compressible chamber confronting the surrounding shoulder portion, and an accommodation chamber confronting the rear surrounding edge portion. The inner barrier wall surface has a grip segment.

The tubular needle seat includes a hub portion disposed to fix the needle cannula therein. The hub portion has a surrounding front end wall, a surrounding gripped portion and an anchoring portion. The surrounding gripped portion is retained at the grip segment by virtue of a second frictional force generated therebetween when the surrounding front end wall abuts against the surrounding abutment wall and when the needle cannula is disposed outwardly of the forward opening.

The plunger is movable in the accommodation chamber, and includes a plunger body and a seal member. The plunger body includes a top end wall disposed movably to abut against the rear surrounding edge portion of the tubular barrier member, and a bottom end wall extending outwardly of the rearward opening so as to be manually operable. The top end wall has an inner peripheral edge portion surrounding the axis to define a cavity therein. The cavity extends along the axis and towards the bottom end wall to contain a fluid at a reduced pressure. The seal member is disposed to be sealingly attached to the inner peripheral edge portion along a sealing line so as to trap the fluid in the cavity.

Other features and advantages of the present invention will become apparent in the following detailed description of the preferred embodiments of the invention, with reference to the accompanying drawings, in which:

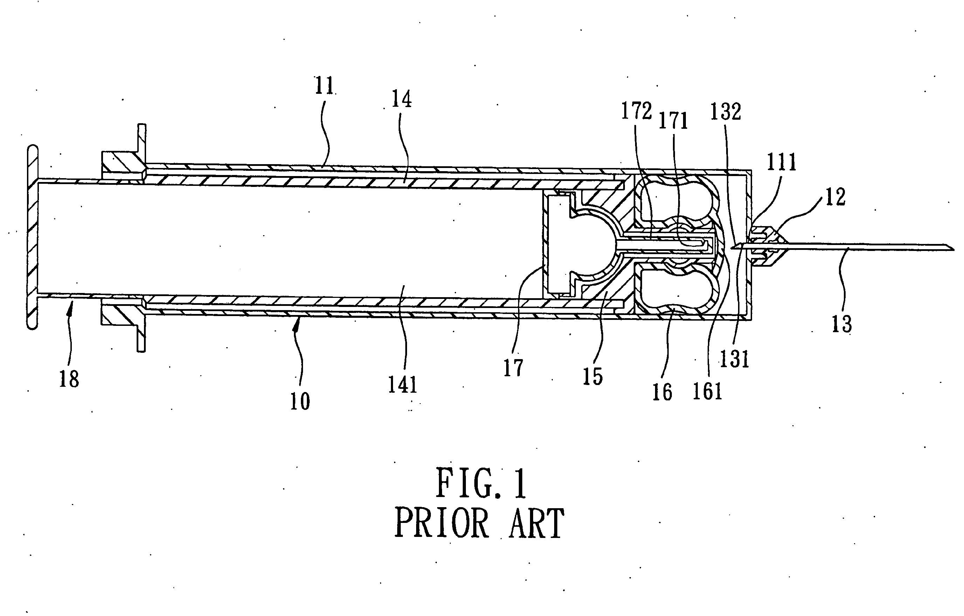

Fig. 1 is a sectional view of a conventional disposable syringe during use;

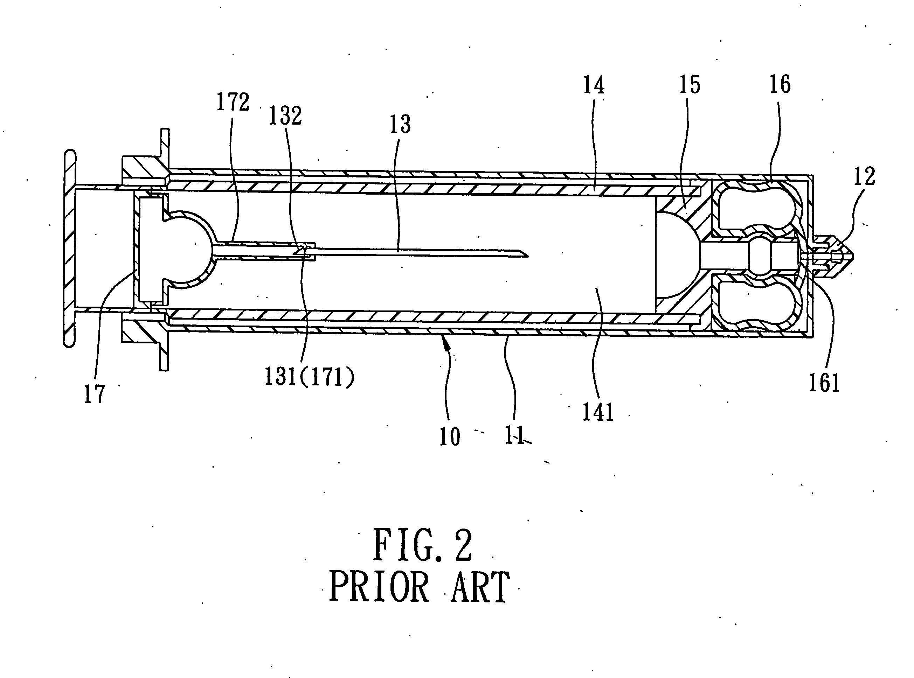

Fig. 2 shows the conventional disposable syringe of Fig. 1 after use, showing a needle cannula in a retracted state;
Fig. 3 is an exploded sectional view of a first preferred embodiment of a disposable syringe according to this invention;
Fig. 4 is a sectional view of the first preferred embodiment during use;
Fig. 5 is a sectional view showing a portion of the first preferred embodiment in detail;
Fig. 6 is a sectional view showing the portion of the first preferred embodiment after use;
Fig. 7 is a sectional view of the first preferred embodiment, showing a needle cannula in a retracted state;
Fig. 8 is a cross-sectional view of a barrel shown in Fig. 5, taken along lines 8-8 thereof;
Fig. 9 is a sectional view of a second preferred embodiment of the disposable syringe according to this invention;
Fig. 10 is a sectional view of the second preferred embodiment, showing a needle cannula in a retracted state;
Fig. 11 is a sectional view of a third preferred embodiment of the disposable syringe according to this invention;
Fig. 12 is a sectional view showing a portion of the third preferred embodiment in detail;
Fig. 13 is a sectional view of the third preferred embodiment, showing a needle cannula in a retracted state;
Fig. 14 is a sectional view of a fourth preferred embodiment of the disposable syringe according to this invention;
Fig. 15 is a sectional view of the fourth preferred embodiment, showing a needle cannula in a retracted state;
Fig. 16 is a sectional view of a fifth preferred embodiment of the disposable syringe according to this invention;
Fig. 17 is a fragmentary sectional view of a sixth preferred embodiment of the disposable syringe according to this invention;
Fig. 18 is a fragmentary sectional view of a seventh preferred embodiment of the disposable syringe according to this invention;
Fig. 19 is a fragmentary sectional view of an eighth preferred embodiment of the disposable syringe according to this invention;
Fig. 20 is a sectional view of a ninth preferred embodiment of the disposable syringe according to this invention; and
Fig. 21 is a fragmentary sectional view showing an alternate embodiment of a sealing member of a plunger of the disposable syringe of this invention.

Before the present invention is described in greater detail, it should be noted that same reference numerals have been used to denote like elements throughout the specification.

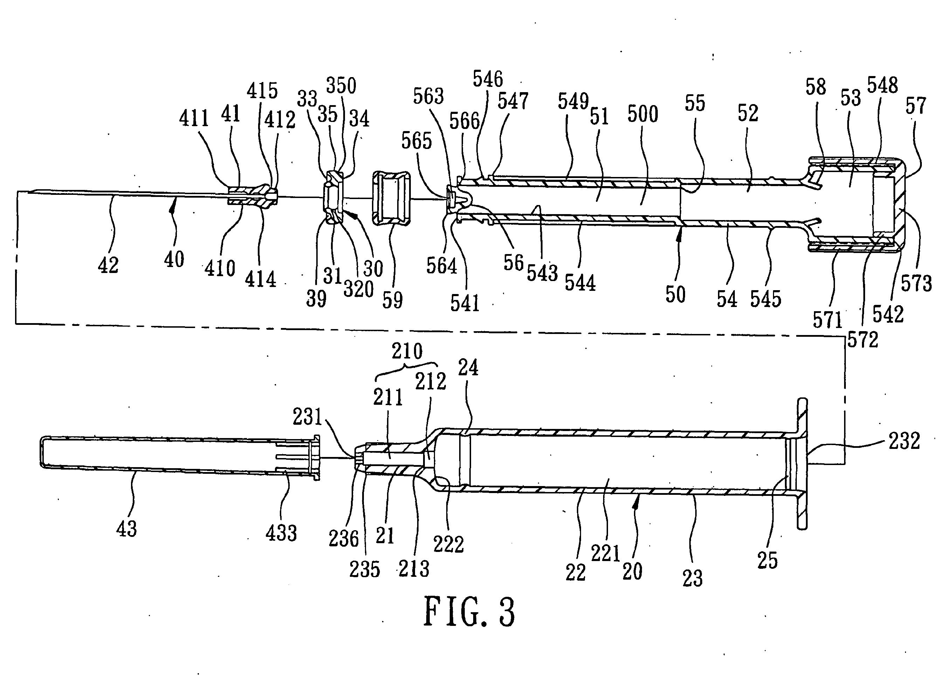

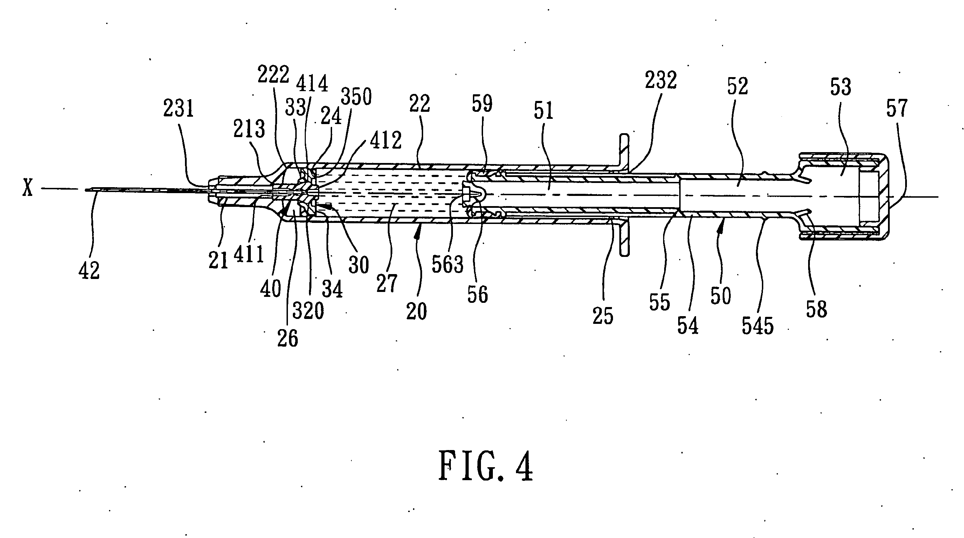

Referring to Figs. 3, 4 and 5, the first preferred embodiment of the disposable syringe according to the present invention is shown to comprise a barrel 20, a tubular barrier member 30, a plunger 50, and a needle assembly 40 including a needle cannula 42, a tubular needle seat 41 and a tip protector 43.

The barrel 20 has an inner surrounding barrel wall surface which surrounds an axis (X) and which confines a passage. The passage has rearward and forward openings 232,231 which are disposed opposite to each other in a longitudinal direction parallel to the axis (X). The inner surrounding barrel wall surface includes a larger-diameter segment 22 and a smaller-diameter segment 21 which confine rear and front passageways 221,210, respectively, and which are disposed proximate to the rearward and forward openings 232,231, respectively, to form a surrounding shoulder portion 222 therebetween. The smaller-diameter segment 21 includes a front surrounding region 211, and a rear surrounding region 212 which is proximate to the surrounding shoulder portion 222 and which is of a larger dimension than that of the front surrounding region 211 so as to form a surrounding abutment wall 213 therebetween. The larger-diameter segment 22 includes proximate and distal surrounding regions opposite to each other in the longitudinal direction and respectively proximate to and distal from the surrounding shoulder portion 222 . The proximate surrounding region has a retaining area which is spaced apart from the surrounding shoulder portion 222 in the longitudinal direction. In this embodiment, an annular first retaining protrusion 24 is formed on the proximate surrounding region in the retaining area. An annular injection limiting protrusion 25 is formed on the distal surrounding region adjacent to the rearward opening 232. An outer surrounding barrel wall surface 23 of the barrel 20 has a rib portion 235 which extends in the longitudinal direction and which is disposed at the front surrounding region 211 adjacent to the forward opening 231.

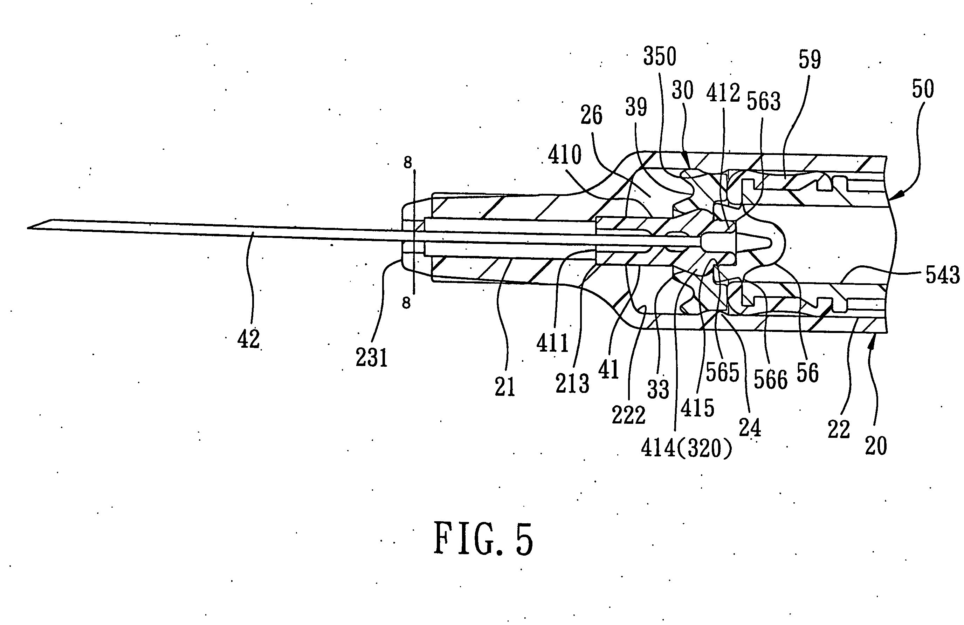

The tubular barrier member 30 includes front and rear surrounding edge portions 33,34 opposite to each other in the longitudinal direction and which includes inner and outer barrier wall surfaces 31,35 opposite to each other and surrounding the axis (X). The outer barrier wall surface 35 is formed with an annular first retaining groove 350 such that in a position of use, the first retaining protrusion 24 is retained in the first retaining groove 350 in a water-tight manner so as to generate a first frictional force therebetween, thereby partitioning the rear passageway 221 into a compressible chamber 26 which confronts the surrounding shoulder portion 222, and an accommodation chamber 27 which confronts the rear surrounding edge portion 34. The inner barrier wall surface 31 has a second retaining groove 320 which serves as a grip segment. As shown in Fig. 5, the front surrounding edge portion 33 has a surrounding groove 39 surrounding the axis (X) such that the inner barrier wall surface 31 is more deformable in radial directions relative to the axis (X). In addition, the inner barrier wall surface 31 is divergent from the second retaining groove 320 to the front surrounding edge portion 33 so as to prevent friction contact between the inner barrier wall surface 31 and a hub portion 410.

The tubular needle seat 41 includes the hub portion 410 which is disposed to fix the needle cannula 42 therein, and which has a surrounding front end wall 411 that extends radially relative to the axis (X). An annular second retaining protrusion 414 is formed on an end of the hub portion 410 extending in the longitudinal direction away from the surrounding front end wall 411 to form a surrounding gripped portion, which is retained in the second retaining groove 320 to generate a second frictional force when the surrounding front end wall 411 abuts against the surrounding abutment wall 213 and when the needle cannula 42 is disposed outwardly of the forward opening 231. An anchoring portion 412 extends from the second retaining protrusion 414 in the longitudinal direction and away from the hub portion 410, and has an annular third retaining protrusion 415 formed thereon (see Fig. 5).

The tip protector 43 has a sleeve end 433 which is disposed to sleeve on the outer surrounding barrel wall surface 23. The sleeve end 433 includes a groove portion which mates with the rib portion 235 to result in a splined engagement between the tip protector 43 and the outer surrounding barrel wall surface 23, thereby ensuring secure shielding of the needle cannula 42. Moreover, as the sleeve end 433 of the tip protector 43 is provided with the groove portion to engage the rib portion 235 on the outer surrounding barrel wall surface 23, the sleeve end 433 has a relatively large inner diameter, thereby enhancing safety during sleeving of the tip protector 43 onto the outer surrounding barrel wall surface 23.

The plunger 50 is disposed to be slidable in the accommodation chamber 27, and includes a plunger body 54, an end cap 57 and a seal member 56.

The plunger body 54 includes an open top end wall 541 which is disposed movably to engage against the rear surrounding edge portion 34 of the tubular barrier member 30, and an open bottom end wall 542 which is disposed opposite to the top end wall 541 in the longitudinal direction and which extends outwardly of the rearward opening 232. The plunger body 54 has an inner peripheral edge portion 543 which surrounds the axis (X) and which defines a cavity 500 therein. The cavity 500 extends along the axis (X) from the top end wall 541 to the bottom end wall 542 to contain a fluid at a reduced pressure. Preferably, the cavity 500 is in a substantially vacuum state. In this embodiment, the cavity 500 includes a smaller-diameter front cavity segment 51 and a larger-diameter rear cavity segment 52 which are disposed proximate to the top and bottom end walls 541,542, respectively, to form a shoulder portion 55 therebetween. Preferably, an enlarged cavity segment 53 is disposed rearwardly of the rear cavity segment 52 and outwardly of the rearward opening 232 so as to be manually operable. A plurality of rib plates 549 are formed on an outer peripheral wall surface 544 of the plunger body 54 at the front cavity segment 51, and are flush with the outer peripheral wall surface 544 at the rear cavity segment 52. An annular injection limiting protrusion 545 is formed on the outer peripheral wall surface 544 at the rear cavity segment 52 adjacent to the enlarged cavity segment 53, and is disposed to abut against the injection limiting protrusion 25 when the top end wall 541 reaches the rear surrounding edge portion 34 so as to indicate completion of an injection course.

Moreover, an annular anchoring protrusion 546 and an annular retaining flange 547 are formed adjacent to the top end wall 541. A surrounding sealing ring 59 is made of a deformable material, and is sleeved retainingly over the outer peripheral wall surface 544 adjacent to the top end wall 541 by means of the anchoring protrusion 546 and the retaining flange 547.

The end cap 57 includes an outer surrounding wall 571 which engages threadedly a threaded segment 548 of the outer peripheral wall surface 544, an inner surrounding wall 572 which is attached to the inner peripheral edge portion 543, and an end cap wall 573 which is connected to the outer and inner surrounding walls 571,572 and which covers an opening in the bottom end wall 542 in an air-tight sealing manner.

A plurality of barrier ribs 58 are formed between the rear cavity segment 52 and the enlarged cavity segment 53. Each barrier rib 58 extends from the inner peripheral edge portion 543 radially and toward the axis (X).

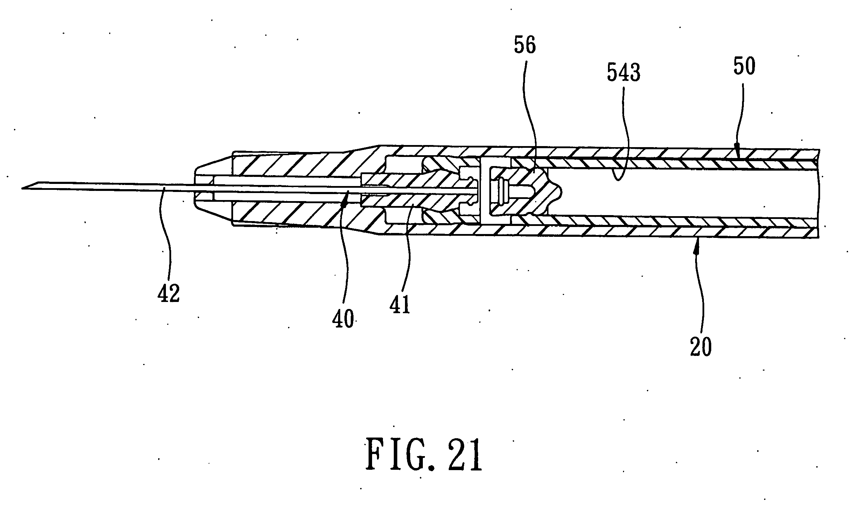

The seal member 56 is disposed to be sealingly attached to the inner peripheral edge portion 543 at the top end wall 541 along an annular sealing line 566 so as to trap the fluid in the cavity 500 so as to maintain the reduced pressure (the substantially vacuum state in this embodiment). In the embodiment shown in Fig. 3, the seal member 56 is formed on the inner peripheral edge portion 543. Alternately, referring to Fig. 21, the seal member 56 is disposed to be press-fitted to the inner peripheral edge portion 543 so as to form a sealing region. The seal member 56 has an engaging recess 563 which confronts the anchoring portion 412 and which extends in the longitudinal direction, and a third retaining groove 565 which is disposed in a front side of the engaging recess 563 and which extends in a radial direction relative to the axis (X), as shown in Fig. 5.

In use, the plunger 50 is pressed forwardly by a pushing force applied to the end cap 57 to push the surrounding sealing ring 59 to reach the rear surrounding edge portion 34 until the injection limiting protrusion 545 abuts against the injection limiting protrusion 25 to indicate the completion of the injection course. In this state, the anchoring portion 412 is retained in the engaging recess 563 by engagement of the third retaining protrusion 415 and the third retaining groove 565, thereby resulting in securing the needle seat 41 to the seal member 56.

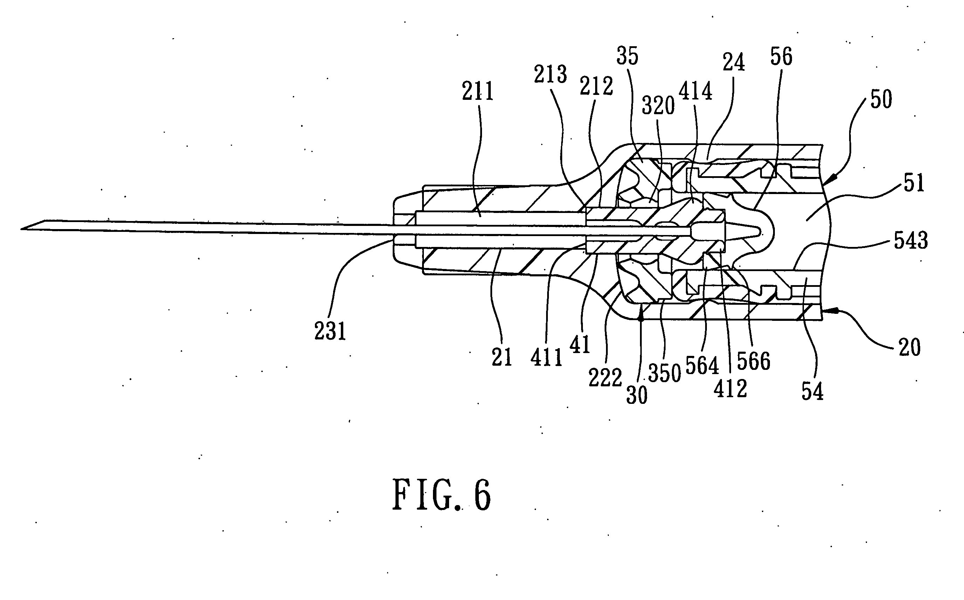

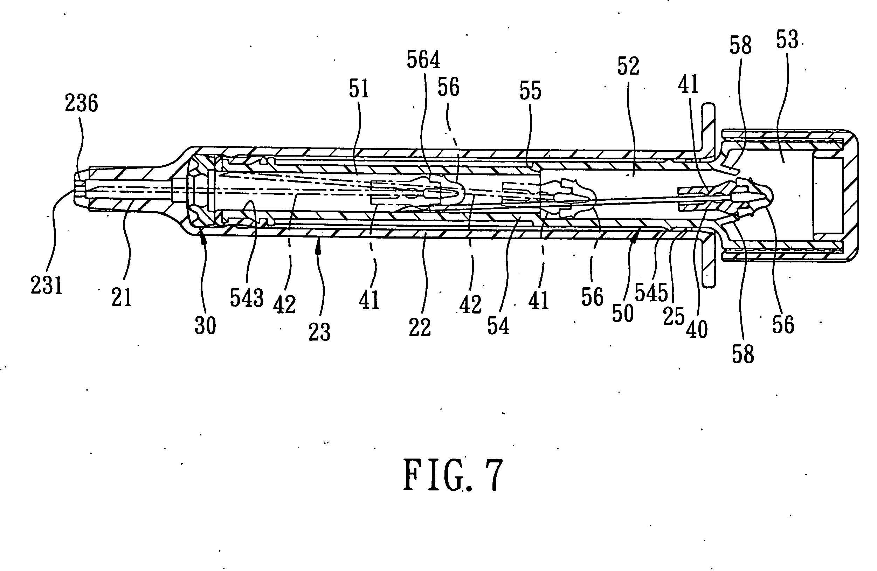

Subsequently, with reference to Figs. 5 and 6, when a pushing force greater than the first and second frictional forces, i. e. frictional forces generated between the first retaining protrusion and groove 24,350 and between the second retaining protrusion and groove 414,320, is further applied to the end cap 57, the tubular barrier member 30 is pushed to move towards the surrounding shoulder portion 222 to force the second retaining groove 320 to disengage from the second retaining protrusion 414, so that the anchoring portion 412, which remains fixedly in place due to abutment of the surrounding front end wall 411 against the surrounding abutment wall 213, is exposed to and is impacted by an impact force from the seal member 56 along the axis (X) that the sealing line 566 is ruptured. Thus, the seal member 56 is released from the plunger body 54, and the needle seat 41 and the needle cannula 42, together with the seal member 56, are suctioned into the cavity 500 by a suction force resulting from a pressure difference between the ambient atmosphere and the reduced pressure in the cavity 500, as shown in Fig. 7. Note that the seal member 56 has an outer surrounding seal surface 564 which is configured to be kept in slidable contact and air-tight engagement with the inner peripheral edge portion 543 when the seal member 56 is suctioned in the front cavity segment 51. Moreover, by virtue of the shoulder portion 55 and the barrier ribs 58, the assembly of the needle seat 41, the needle cannula 42 and the seal member 56 can be trapped in the cavity 500.

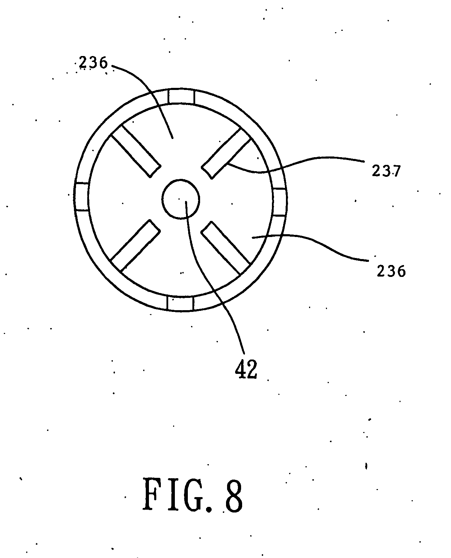

In addition, as shown in Fig. 8, the inner surrounding barrel wall surface of the barrel 20 is spaced apart from the needle cannula 42 in radial directions at the forward opening 231 to define a surrounding clearance therebetween. In particular, a plurality of ribs 237 are disposed on the inner surrounding barrel wall surface and are angularly displaced from one another. Each rib 237 extends toward the needle cannula 42 while forming an air duct 236 between two adjacent ones of the ribs 237 to communicate the passage of the barrel 20 with the ambient atmosphere. As such, the air ducts 236 can facilitate inflow of the ambient air into the cavity 500.

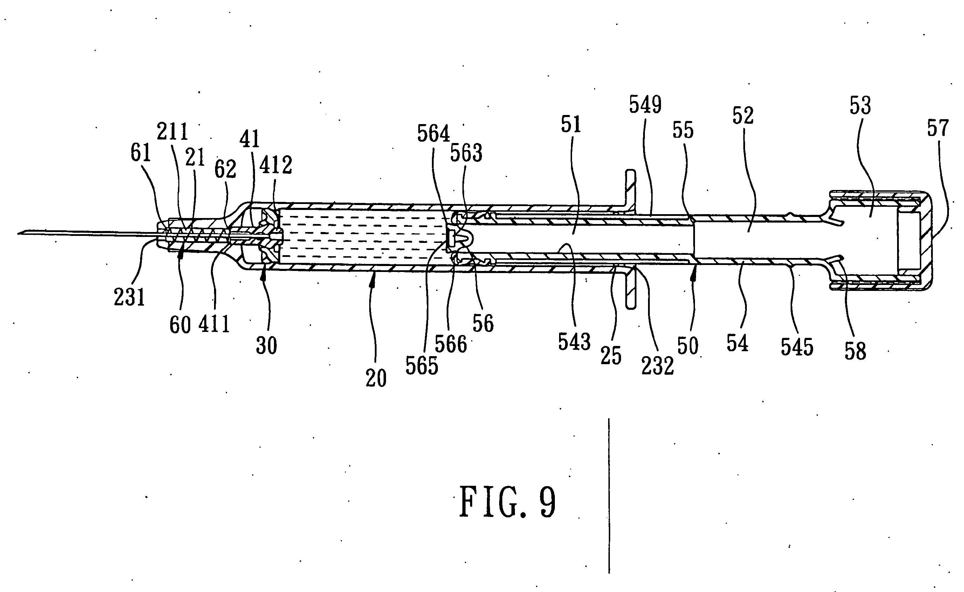

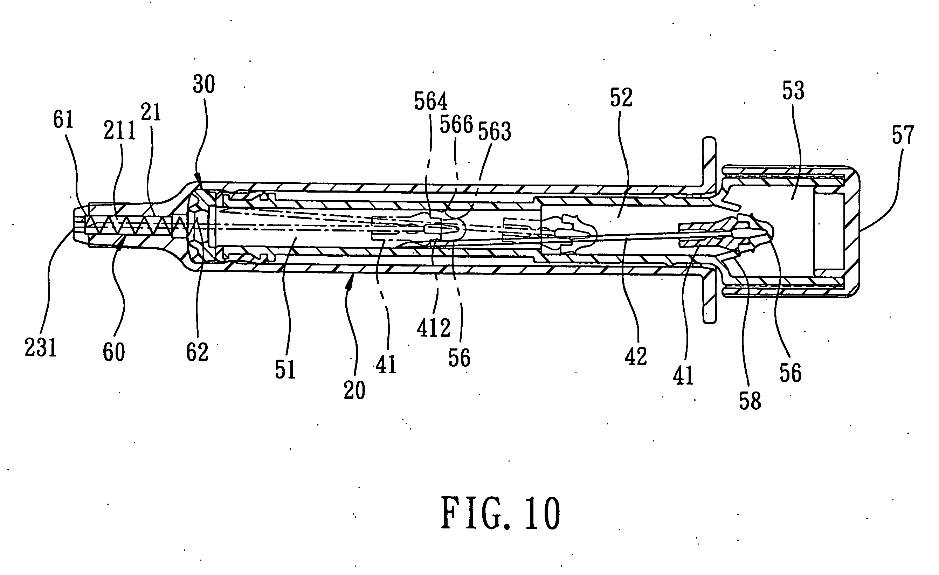

Referring to Figs. 9 and 10, the second preferred embodiment of the disposable syringe according to this invention is shown to be similar to the aforesaid embodiment in construction. The disposable syringe of this embodiment further includes a biasing member 60, such as a compression spring, which is disposed in the front surrounding region 211 to bias the needle seat 41 toward the seal member 56. The biasing member 60 has two ends 61, 62 abutting against the inner surrounding barrel wall surface at the forward opening 231 and the surrounding front end wall 411, respectively, so as to increase the impact force to facilitate retraction action of the suctioned assembly into the cavity 500.

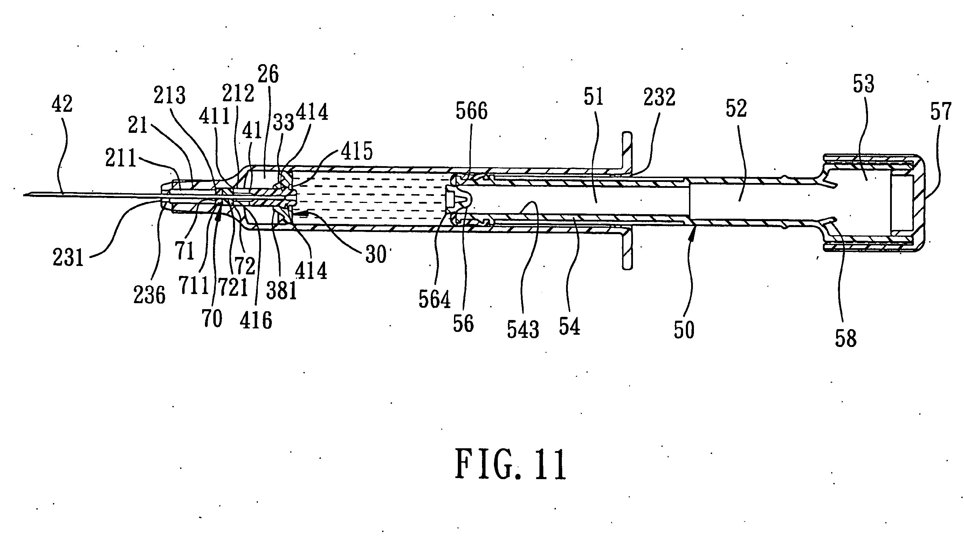

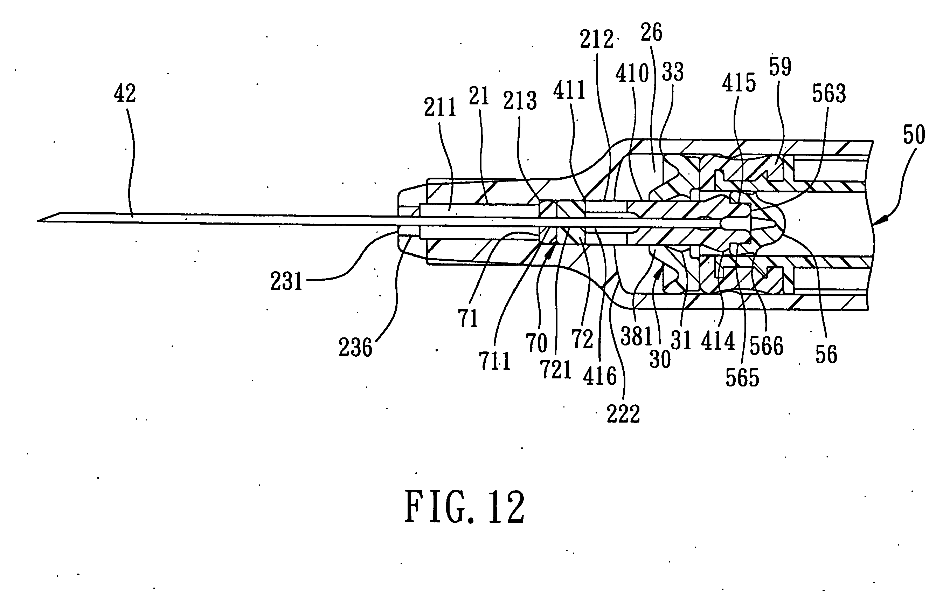

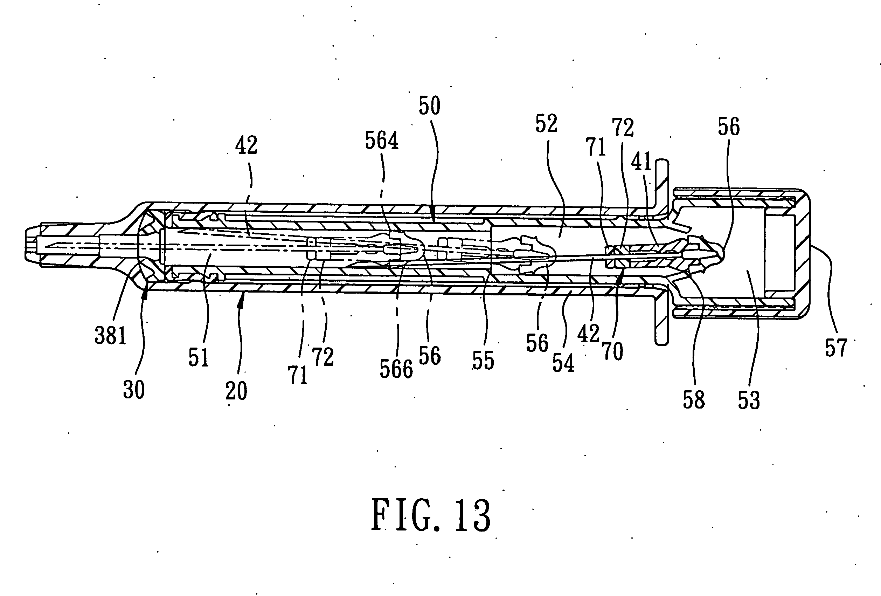

Referring to Figs. 11, 12 and 13, the third preferred embodiment of the disposable syringe according to this invention is shown to be similar to the first preferred embodiment in construction. In this embodiment, the surrounding front end wall 411 includes a sealing member 70 which abuts against the surrounding abutment wall 213 and which is in air-tight engagement with the rear surrounding region 212 so as to maintain air-tightness of the compressible chamber 26. The sealing member 70 includes an elastomeric plate 71 and an elastomeric ring 72 which abut against each other and which have axial holes 711, 721 that extend along the axis (X) to engage fittingly the needle cannula 42 therein. In addition, the inner barrier wall surface 31 of the tubular barrier member 30 further extends from the front surrounding edge portion 33 to have a deformable sealing portion 381 which is in air-tight engagement with the hub portion 410 of the tubular needle seat 41.

The compressible chamber 26 is filled with fluid. The hub portion 410 of the tubular needle seat 41 has a plurality of through holes 416 which are formed therethrough to be in fluid communication with the compressible chamber 26 and which extend in the longitudinal direction. As such, when the tubular barrier member 30 is moved towards the surrounding shoulder portion 222, the fluid is compressed to flow into the through holes 416, thereby helping force the anchoring portion 412 to move toward the seal member 56 so as to increase the impact force.

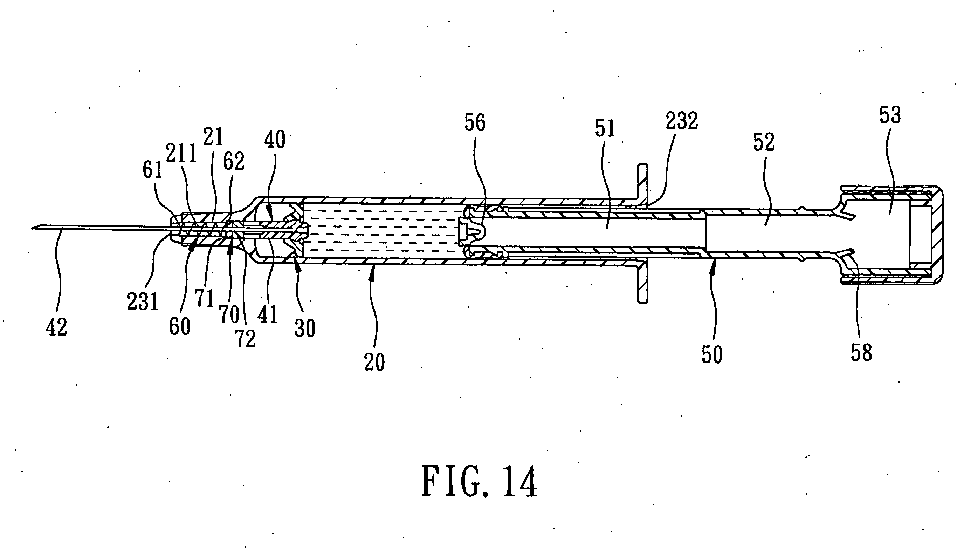

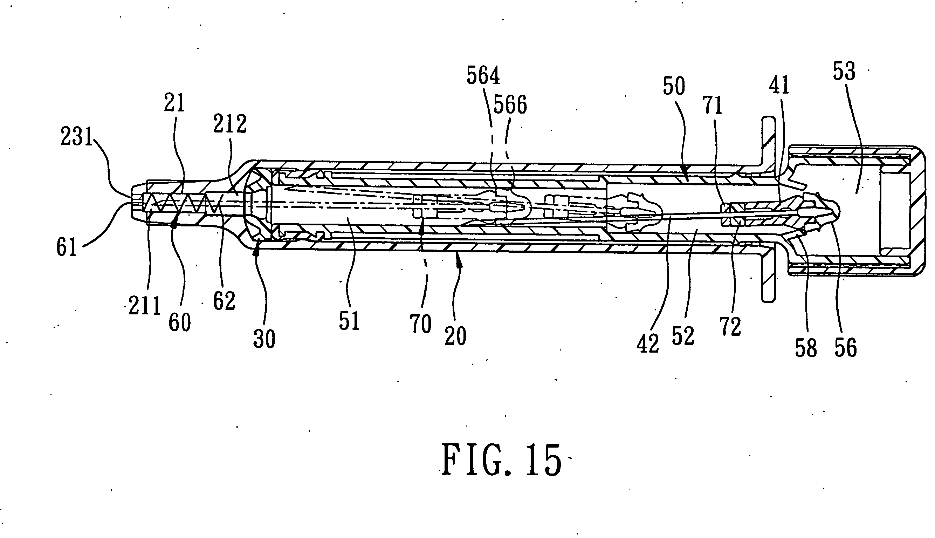

Referring to Figs. 14 and 15, the fourth preferred embodiment of the disposable syringe according to this invention is shown to be similar to the third preferred embodiment in construction, and further includes a biasing member 60 similar to that of the second preferred embodiment to bias the needle seat 41 toward the seal member 56. The biasing member 60 has two ends 61,62 abutting against the inner surrounding barrel wall surface at the forward opening 231 and the elastomeric plate 71 of the sealing member 70, respectively, so as to increase the impact force to facilitate retraction action of the suctioned assembly into the cavity 500.

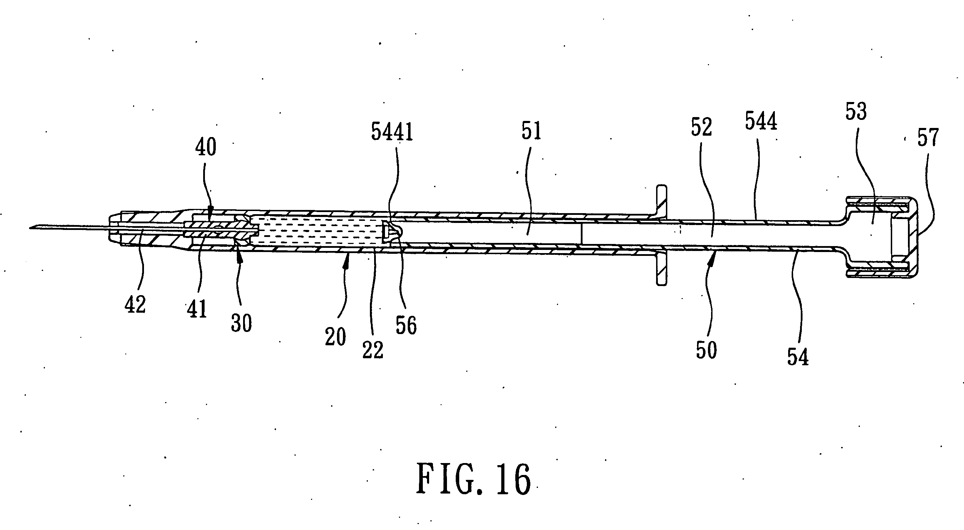

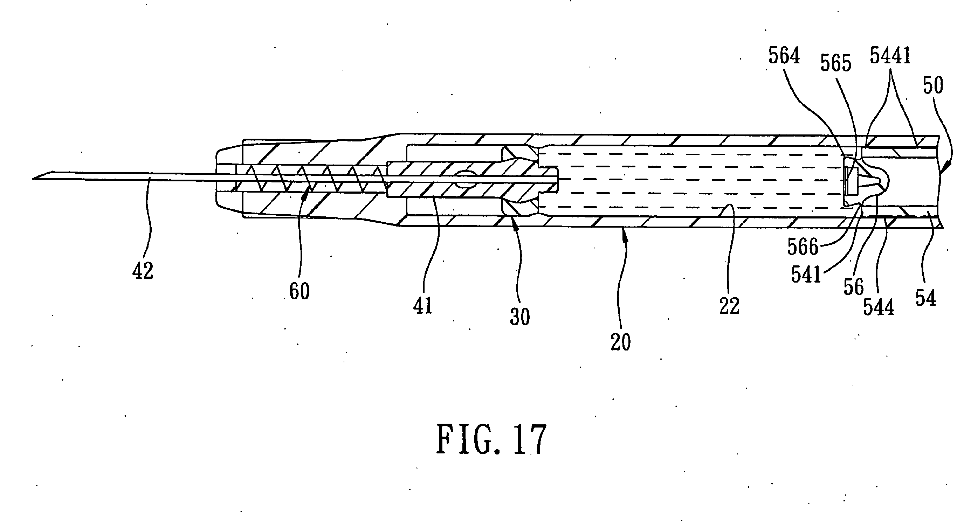

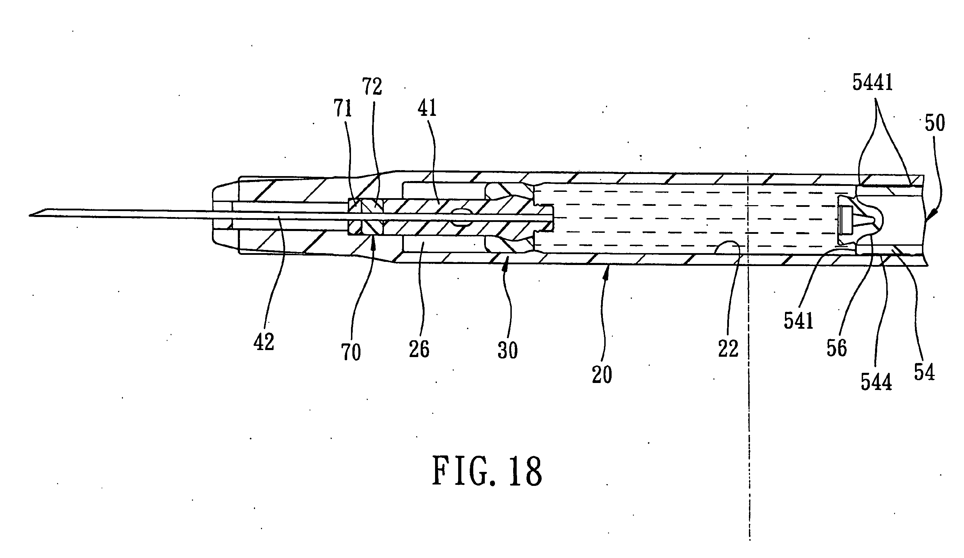

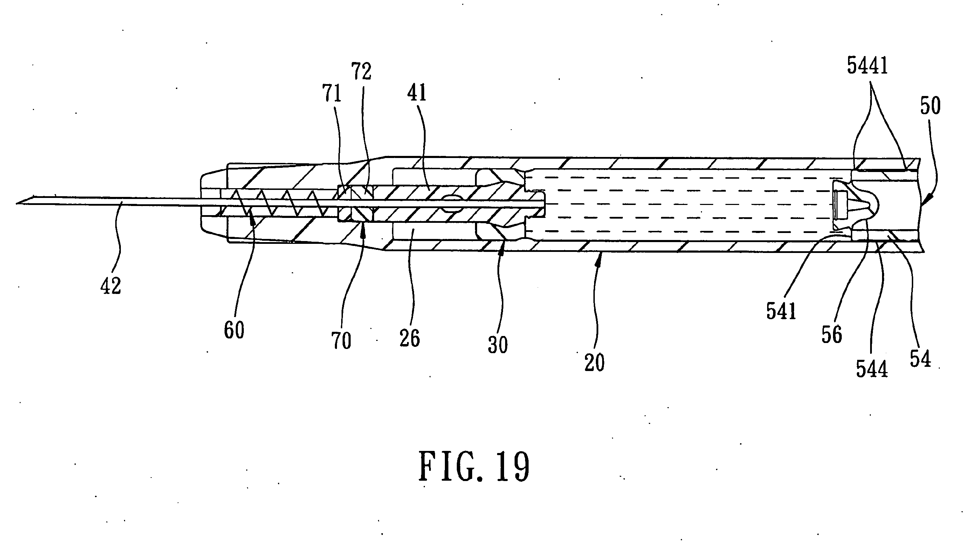

Referring to Figs. 16, 17, 18 and 19, the fifth, sixth, seventh and eighth preferred embodiments of the disposable syringe according to this invention are shown to be respectively similar to the first, second, third and fourth preferred embodiment in construction, but are used for an extremely small injection volume, such as 1 ml. Thus, the barrel 20, the tubular barrier member 30, the needle assembly 40, the plunger 50 and the biasing member 60 are comparatively smaller. In addition, in stead of a separate surrounding sealing ring 59, the surrounding sealing ring in these embodiments includes a plurality of ring portions 5441 which are formed integrally with the outer peripheral wall surface 544 adjacent to the top end wall 541 for slidable and air-tight engagement with the larger-diameter segment 22. Therefore, the annular anchoring protrusion 546 and the annular retaining flange 547 (see Fig. 3) may be eliminated.

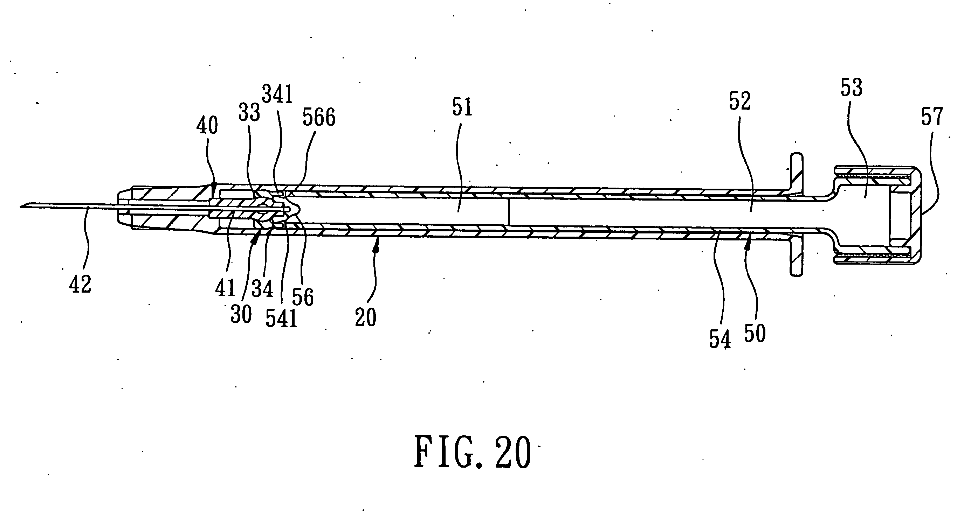

As shown in Fig. 20, the ninth preferred embodiment of the disposable syringe according to this invention is shown to be similar to the fifth preferred embodiment in construction. In this embodiment, the tubular barrier member 30 further includes an annular flange 341 which extends from the rear surrounding edge portion 34 and which is configured to be inserted into a clearance between the top end wall 541 of the plunger body 54 and the rear surrounding edge portion 34, thereby preventing trapping of medicine within the clearance.

As illustrated, the disposable syringe of this invention has the following advantages:
1. The suction of the tubular needle seat 41 and the needle cannula 42 occurs after the top end wall 541, the surrounding sealing ring 59, the seal member 56 and the tubular barrier member 30 are in tight contact with the tubular needle seat 41. Thus, the clearance may not exist therebetween to minimize trapping of medicine or blood within the barrel 20 after use.
2. By virtue of the first and second frictional forces and the abutment of the surrounding front end wall 411 against the surrounding abutment wall 213, the tubular needle seat 41 can be retained firmly in the retaining area during use. In addition, once the tubular needle seat 41 is released from the tubular barrier member 30 by a pushing force applied to the tubular barrier member 30, the tubular needle seat 41 can be suctioned smoothly and easily into the cavity 500.
3. After use, the needle seat 41 and the needle cannula 42 can be retracted into the cavity 500 of the plunger body 54 without the application of a pulling force to the plunger 50. Thus, the plunger body 54 can remain in the rear passageway 221 of the barrel 20, thereby facilitating the disposal of the disposable syringe.

## Claims

1. A disposable syringe including:
a needle cannula (42),
a tubular needle seat (41) including a hub portion (410) which is disposed to fix said needle cannula (42) therein,
a barrel (20) having an inner surrounding barrel wall surface which surrounds an axis (X) and which confines a passage, said passage having rearward and forward openings (232,231) which are disposed opposite to each other in a longitudinal direction parallel to the axis (X), and
a plunger (50) which, in the use position, is disposed to be movable in said passage, **characterized in that:**
said inner surrounding barrel wall, surface of said barrel (20) including a larger-diameter segment (22) and a smaller-diameter segment (21) which confine rear and front passageways (221,210), respectively, and which are disposed proximate to said rearward and forward openings (232,231), respectively, to form a surrounding shoulder portion (222) therebetween, said smaller-diameter segment (21) including a front surrounding region (211) and a rear surrounding region (212) which is proximate to said surrounding shoulder portion (222) and which is of a larger dimension than that of said front surrounding region (211) so as to form a surrounding abutment wall (213) therebetween, said larger-diameter segment (22) including proximate and distal surrounding regions opposite to each other in the longitudinal direction and respectively proximate to and distal from said surrounding shoulder portion (222), said proximate surrounding region having a retaining area which is spaced apart from said surrounding shoulder portion (222) in the longitudinal direction;
a tubular barrier member (30) which includes front and rear surrounding edge portions (33,34) opposite to each other in the longitudinal direction and which includes inner and outer barrier wall surfaces (31,35) opposite to each other and surrounding the axis (X) , said outer barrier wall surface (35) , in a position of use, being retained at said retaining area by virtue of a first frictional force generated therebetween while in water-tight engagement with said proximate surrounding region, thereby partitioning said rear passageway (221) into a compressible chamber (26) which confronts said surrounding shoulder portion (222), and an accommodation chamber (27) which confronts said rear surrounding edge portion (34), said inner barrier wall surface (31) having a grip segment; said hub portion (410) of said tubular needle seat (41) having a surrounding front end wall (411) extending radially relative to the axis (X), said tubular needle seat (41) further including
a surrounding gripped portion which extends from said hub portion (410) in the longitudinal direction and away from said surrounding front end wall (411), and which is retained at said grip segment by virtue of a second frictional force generated between said surrounding gripped portion and said grip segment when said surrounding front end wall (411) abuts against said surrounding abutment wall (213) and when said needle cannula (42) is disposed outwardly of said forward opening (231), and
an anchoring portion (412) extending from said surrounding gripped portion in the longitudinal direction and away from said hub portion (410); said plunger (50) including
a plunger body (54) which includes a top end wall (541) disposed movably to engage against said rear surrounding edge portion (34) of said tubular barrier member (30), and a bottom end wall (542) disposed opposite to said top end wall (541) in the longitudinal direction and extending outwardly of said rearward opening (232) so as to be manually operable, said top end wall (541) having an inner peripheral edge portion (543) which surrounds the axis (X) and which defines a cavity (500) therein, said cavity (500) extending along the axis (X) and towards said bottom end wall (542) to contain a fluid at a reduced pressure, and
a seal member (56) disposed to be sealingly attached to said inner peripheral edge portion (543) along a sealing line (566) so as to trap said fluid in said cavity (500), said sealing line (566) being configured such that when a pushing force greater than the first and second frictional forces, is manually applied to said bottom end wall (542) to move top end wall (541) to engage against said rear surrounding edge portion (34) of said tubular barrier member (30) , said tubular barrier member (30) is pushed to move towards said surrounding shoulder portion (222) to force said grip segment to disengage from said surrounding gripped portion, so that said anchoring portion (412), which remains fixedly in place due to abutment of said surrounding front end wall (411) against said surrounding abutment wall (213), is exposed to and is impacted by an impact force from said seal member (56) along the axis (X) so that said sealing line (566) is ruptured, thereby releasing said seal member (56) from said plunger body (54) , and thereby permitting said tubular needle seat (41) to be forced into engagement with said seal member (56) to be subsequently suctioned into said cavity (500) due to a pressure difference between the ambient atmosphere and the reduced pressure.

2. The disposable syringe of Claim 1, **characterized in that** said barrel (20) has an outer surrounding barrel wall surface (23) which has a rib portion (235) extending in the longitudinal direction and disposed at said front surrounding region (211) adjacent to said forward opening (231), said disposable syringe further comprising a tip protector (43) which has a sleeve end (433) disposed to sleeve on said outer surrounding barrel wall surface (23), said sleeve end (433) including a groove portion which mates with said rib portion (235) to result in a splined engagement between said tip protector (43) and said outer surrounding barrel wall surface (23), thereby ensuring secure shielding of said needle cannula (42).

3. The disposable syringe of Claim 1, **characterized in that** one of said proximate surrounding region in said retaining area and said outer barrier wall surface (35) is formed with a first retaining groove (350), and the other one of said proximate surrounding region in said retaining area and said outer barrier wall surface (35) is formed with a first retaining protrusion (24) which engages retainingly said first retaining groove (350) to generate the first frictional force when said outer barrier wall surface (35) is in the position of use; one of said grip segment of said inner barrier wall surface (31) and said surrounding gripped portion of said tubular needle seat (41) is a second retaining groove (320), and the other one of said grip segment of said inner barrier wall surface (31) and said surrounding gripped portion of said tubular needle seat (41) is a second retaining protrusion (414) which engages retainingly said second retaining groove (320) to generate the second frictional force when said surrounding front end wall (411) abuts against said surrounding abutment wall (213).

4. The disposable syringe of Claim 3, **characterized in that** said surrounding front end wall (411) includes a sealing member (70) which is in air-tight engagement with said rear surrounding region (212) so as to maintain air-tightness of said compressible chamber (26), said compressible chamber (26) being filled with a fluid, said hub portion (410) of said tubular needle seat (41) having a plurality of through holes (416) which are distal from said surrounding front end wall (411) in the longitudinal direction, which are formed therethrough to be in fluid communication with said compressible chamber (26), and which extend in the longitudinal direction such that when said tubular barrier member (30) is moved towards said surrounding shoulder portion (222) , said fluid is forced to flow into said through holes (416), thereby enforcing said anchoring portion (412) to move toward said cavity (500) so as to increase the impact force.

5. The disposable syringe of Claim 4, **characterized in that** said sealing member (70) is made from an elastomeric material, and has an axial hole (711,721) extending along the axis (X) to engage fittingly said needle cannula (42) therein.

6. The disposable syringe of Claim 4, **characterized in that** said inner barrier wall surface (33) is formed with a deformable sealing portion (381) which is in air-tight engagement with said hub portion (410) of said tubular needle seat (41).

7. The disposable syringe of Claim 1 or 4, further **characterized by** a biasing member (60) disposed in said front surrounding region (211) to bias said tubular needle seat (41) toward said seal member (56).

8. The disposable syringe of Claim 3, **characterized in that** said front surrounding edge portion has a surrounding groove (39) surrounding the axis (X) so that said inner barrier wall surface (31) more is deformable in radial directions relative to the axis (X).

9. The disposable syringe of Claim 3, **characterized in that** said inner barrier wall surface (31) is divergent from said grip segment to said front surrounding edge portion (33) to prevent friction contact between said inner barrier wall surface (33) and said hub portion (410) of said tubular needle seat (41) when said tubular needle seat (41) is suctioned into said cavity (500).

10. The disposable syringe of Claim 1, **characterized in that** said plunger body (54) has a surrounding sealing ring (59) which is disposed adjacent to said top end wall (541), which surrounds the axis (X), and which is slidable on and in air-tight engagement with said larger-diameter segment (22) of said inner surrounding barrel wall surface.

11. The disposable syringe of Claim 1, **characterized in that** said seal member (56) has an engaging recess (563) which confronts said anchoring portion (412) and which extends in the longitudinal direction so as to engage said anchoring portion (412) when said seal member (56) is released from said plunger body (54), and a third retaining groove (565) which is disposed in said engaging recess (563) and which extends in a radial direction relative to the axis (X) , said anchoring portion (412) having a third retaining protrusion (415) which is disposed to be retained in said third retaining groove (565) when said anchoring portion (412) engages said engaging recess (563) such that said seal member (56), together with said tubular needle seat (41) , is suctioned into said cavity (500).

12. The disposable syringe of Claim 11, **characterized in that** said cavity (500) includes front and rear cavity segments (51,52) which are disposed proximate to said top and bottom end walls (541,542), respectively, and which have smaller and larger inner diameters, respectively, said seal member (56) having an outer surrounding seal surface (564) which is configured to be kept in slidable contact and air-tight engagement with said inner peripheral edge portion (543) when said seal member (56) is suctioned in said front cavity segment (51).

13. The disposable syringe of Claim 12, **characterized in that** said plunger (50) further includes a plurality of barrier ribs (58) , each of which is disposed adjacent to said bottom end wall (542) and which extends from said inner peripheral edge portion (543) radially and toward the axis (X) so as to trap said tubular needle seat (41) when said tubular needle seat (41) is forced towards said bottom end wall (542).

14. The disposable syringe of Claim 12, **characterized in that** said plunger body (54) further includes an outer peripheral wall surface (544) , and a plurality of rib plates (549) which are formed on said outer peripheral wall surface (544) at said front cavity segment (51) , and which are flush with said outer peripheral wall surface (544) at the rear cavity segment (52).

15. The disposable syringe of Claim 1, **characterized in that** said inner surrounding barrel wall surface is spaced apart from said needle cannula (42) in radial directions relative to the axis (X) at said forward opening (231) to define a surrounding clearance therebetween.

16. The disposable syringe of Claim 15, further **characterized by** a plurality of ribs (237) which are disposed on said inner surrounding barrel wall surface and which are angularly displaced from one another, each of said ribs (237) extending towards said needle cannula (42), an air duct (236) being formed between two adjacent ones of said ribs (237) to communicate said passage with the ambient atmosphere.

17. The disposable syringe of Claim 1, further **characterized by** two injection limiting protrusions (545,25) which are disposed on said plunger body (54) adjacent to said bottom end wall (542) and said larger-diameter segment (22) adj acent to said rearward opening (232) respectively, and which abut against each other when said top end wall (541) reaches said rear surrounding edge portion (34) so as to limit an extent of an injection course.

18. The disposable syringe of Claim 1, **characterized in that** said bottom end wall (542) has an opening which is in fluid communication with said cavity (500), said plunger (50) further including an end cap (57) which is disposed to cover said opening and to be in air-tight engagement with said bottom end wall (542).

## Patentansprüche

1. Wegwerf-Injektionsspritze, die umfasst:
eine Nadelkanüle (42),
einen rohrförmigen Nadelsitz (41), der einen Nabenabschnitt (410) umfasst, der angeordnet ist, um die Nadelkanüle (42) darin zu befestigen,
einen Zylinder (20), der eine innere umlaufende Zylinderwandoberfläche hat, die eine Achse (X) umgibt und einen Durchgang begrenzt, wobei der Durchgang eine vordere und eine hintere Öffnung (232, 231) hat, die zueinander entgegengesetzt in einer zu der Achse (X) parallelen Längsrichtung angeordnet sind, und
einen Kolben (50), der angeordnet ist, um in der Benutzungsstellung in dem Durchgang bewegbar zu sein,
**dadurch gekennzeichnet, dass**
die innere umlaufende Zylinderwandoberfläche des Zylinders (20) einen Abschnitt (22) mit einem größeren Durchmesser und einen Abschnitt (21) mit einem kleineren Durchmesser umfasst, die den hinteren bzw. vorderen Durchlass (221, 210) begrenzen und die unmittelbar neben der hinteren bzw. vorderen Öffnung (232, 231) angeordnet sind, um dazwischen einen umlaufenden Schulterabschnitt (222) zu bilden, wobei der Abschnitt (21) mit kleinerem Durchmesser einen vorderen umlaufenden Bereich (211) und einen hinteren umlaufenden Bereich (212) umfasst, der unmittelbar neben dem umlaufenden Schulterabschnitt (222) liegt und der größer dimensioniert ist als der des vorderen umlaufenden Bereichs (211), um eine umlaufende Anlagewand (213) dazwischen auszubilden, wobei der Abschnitt (22) mit größerem Durchmesser einen unmittelbar benachbarten und einen entfernt umlaufenden Bereich umfasst, die in Längsrichtung zueinander entgegengesetzt liegen und jeweils unmittelbar neben oder entfernt von dem umlaufenden Schulterabschnitt (222) angeordnet sind, wobei der unmittelbar benachbarte umlaufende Bereich einen Haltebereich hat, der von dem umlaufenden Schulterabschnitt (222) in der Längsrichtung beabstandet ist;
ein rohrförmiges Begrenzungsglied (30), das einen vorderen und einen hinteren umlaufenden Randabschnitt (33, 34) umfasst, die zueinander in der Längsrichtung entgegen gesetzt liegen, und das eine innere und eine äußere Begrenzungswandoberfläche (31, 35) umfasst, die zueinander entgegengesetzt liegen und die Achse (X) umlaufen, wobei die äußere Begrenzungswandoberfläche (35) in einer Benutzungsstellung aufgrund einer ersten dazwischen erzeugten Reibungskraft in dem Haltebereich gehalten wird, während sie sich in wasserdichtem Eingriff mit dem unmittelbar benachbarten umlaufenden Bereich befindet, **dadurch** den hinteren Durchfluss (221) in eine komprimierbare Kammer (26), die dem umlaufenden Schulterabschnitt (222) gegenübersteht, und in eine Aufnahmekammer (27) unterteilt, die dem hinteren umlaufenden Randabschnitt (34) gegenübersteht, wobei die innere Begrenzungswandoberfläche (31) einen Greifabschnitt aufweist; wobei der Nabenabschnitt (410) des rohrförmige Nadelsitzes (41) eine umlaufende vordere Endwand (411) aufweist, die sich bezüglich der Achse (X) radial erstreckt,
wobei der rohrförmige Nadelsitz (41) weiter umfasst
einen umlaufenden gegriffenen Abschnitt, der sich von dem Nabenabschnitt (410) in der Längsrichtung und von der umlaufenden vorderen Endwand (411) weg erstreckt, und der an dem Greifabschnitt aufgrund einer zweiten Reibungskraft gehalten wird, die zwischen dem umlaufenden gegriffenen Abschnitt und dem Greifabschnitt erzeugt wird, wenn die umlaufende vordere Endwand (411) an der umlaufenden Anlagewand (213) anliegt und wenn die Nadelkanüle (42) außerhalb der vorderen Öffnung (231) angeordnet ist, und
einen Verankerungsabschnitt (412), der sich von dem umlaufenden gegriffenen Abschnitt in der Längsrichtung und von dem Nabenabschnitt (410) weg erstreckt;
wobei der Kolben (50) umfasst:
einen Kolbenkörper (54), der eine obere Endwand (541) umfasst, die bewegbar angeordnet ist, um an dem hinteren umlaufenden Randabschnitt (34) des rohrförmigen Begrenzungsglieds (30) anzugreifen, und eine untere Endwand (542), die zu der oberen Endwand (541) in der Längsrichtung entgegengesetzt angeordnet ist und sich außerhalb der hinteren Öffnung (232) erstreckt, um manuell betätigbar zu sein, wobei die obere Endwand (541) einen inneren Umfangsrandabschnitt (543) aufweist, der die Achse (X) umläuft und der einen Hohlraum (500) darin bestimmt, wobei der Hohlraum (500) sich entlang der Achse (X) und in Richtung zu der unteren Endwand (542) hin erstreckt, um ein Fluid unter einem verringerten Druck aufzunehmen, und
ein Dichtglied (56), das angeordnet ist, um dichtend an dem inneren Umfangsrandabschnitt (543) entlang einer Dichtlinie (566) angebracht zu sein, um das Fluid in dem Hohlraum (500) einzuschließen, wobei die Dichtlinie (566) derart ausgebildet ist, dass dann, wenn eine Druckkraft, die größer als die erste und zweite Reibungskraft ist, manuell auf die untere Endwand (542) aufgebracht wird, um die obere Endwand (541) zu bewegen, um an dem hinteren umlaufenden Randabschnitt (34) des rohrförmigen Begrenzungsglieds (30) anzugreifen, das rohrförmige Begrenzungsglied (30) derart verschoben wird, dass es sich in Richtung zu dem umlaufenden Schulterabschnitt (222) bewegt, um den Greifabschnitt zu veranlassen, sich von dem umlaufenden gegriffenen Abschnitt zu lösen, so dass der Verankerungsabschnitt (412), der aufgrund der Anlage der umlaufenden vorderen Endwand (411) an der umlaufenden Anlagewand (213) starr an seinem Platz verbleibt, einer von dem Dichtglied (56) ausgehenden Stoßkraft entlang der Achse (X) ausgesetzt ist und von ihr beeinflusst wird, so dass die Dichtlinie (566) unterbrochen wird, wodurch das Dichtglied (56) von dem Kolbenkörper (54) freigegeben wird und wodurch ermöglicht wird, dass der rohrförmige Nadelsitz (41) in Eingriff mit dem Dichtglied (56) gebracht wird, um infolgedessen aufgrund einer Druckdifferenz zwischen der umgebenden Atmosphäre und dem verminderten Druck in den Hohlraum (500) eingesaugt zu werden.

2. Wegwerf-Injektionsspritze nach Anspruch 1, **dadurch gekennzeichnet, dass** der Zylinder (20) eine äußere umlaufende Zylinderwandoberfläche (23) aufweist, die einen Rippenabschnitt (235) hat, der sich in der Längsrichtung erstreckt und an dem vorderen umlaufenden Bereich (211) benachbart der vorderen Öffnung (231) angeordnet ist, wobei die Wegwerf-Injektionsspritze weiter einen Spitzenschutz (43) aufweist, der ein Hülsenende (433) hat, das derart angeordnet ist, dass es die äußere umlaufende Zylinderwandoberfläche (23) umgibt, wobei das Hülsenende (433) einen Nutabschnitt umfasst, der mit dem Rippenabschnitt (235) derart zusammenfasst, dass sich eine Nutverbindung zwischen dem Spitzenschutz (43) und der äußeren umlaufenden Zylinderwandoberfläche (23) ergibt und dabei einen sicheren Schutz der Nadelkanüle (42) sicherstellt.

3. Wegwerf-Injektionsspritze nach Anspruch 1, **dadurch gekennzeichnet, dass** entweder der benachbarte umlaufende Bereich in dem Haltebereich oder die äußere Begrenzungswandoberfläche (35) mit einer ersten Haltenut (350) ausgebildet ist, und dass der jeweils andere von unmittelbar benachbartem umlaufendem Bereich in dem Haltebereich oder äußerer Grenzwandoberfläche (35) mit einem ersten Haltevorsprung (24) ausgebildet ist, der haltend an der ersten Haltenut (350) angreift, um die erste Reibungskraft zu erzeugen, wenn sich die äußere Grenzwandoberfläche (35) in der Benutzungsstellung befindet; wobei entweder der Greifabschnitt der inneren Grenzwandoberfläche (31) oder der umlaufende gegriffene Abschnitt des rohrförmigen Nadelsitzes (41) eine zweite Haltenut (320) ist, und der andere von Greifabschnitt der inneren Grenzwandoberfläche (31) oder umlaufendem gegriffenem Abschnitt des rohrförmigen Nadelsitzes (41) ein zweiter Haltevorsprung (414) ist, der haltend an der ersten Haltenut (320) angreift, um die zweite Reibungskraft zu erzeugen, wenn die umlaufende vordere Endwand (411) an der umlaufenden Anlagewand (213) anliegt.

4. Wegwerf-Injektionsspritze nach Anspruch 3, **dadurch gekennzeichnet, dass** die umlaufende vordere Endwand (411) ein Dichtglied (70) umfasst, das in luftdichtem Eingriff mit dem hinteren umlaufenden Bereich (212) ist, so dass die komprimierbare Kammer (26) luftdicht bleibt, wobei die komprimierbare Kammer (26) mit einem Fluid gefüllt ist, wobei der Nabenabschnitt (410) des rohrförmigen Nadelsitzes (41) eine Vielzahl an Durchgangsöffnungen (416) aufweist, die von der umlaufenden vorderen Endwand (411) in der Längsrichtung entfernt sind, die durch diese hindurch laufend ausgebildet sind, um in Fluidaustausch mit der komprimierbaren Kammer zu stehen, und die sich in der Längsrichtung erstrecken, so dass dann, wenn das rohrförmige Begrenzungsglied (30) zu dem umlaufenden Schulterabschnitt (222) hin bewegt wird, das Fluid dazu gebracht wird, in die Durchgangsöffnungen (416) zu fließen, wodurch erzwungen wird, dass der Verankerungsabschnitt (412) sich in Richtung zu dem Hohlraum (500) bewegt, so dass die Stoßkraft zunimmt.

5. Wegwerf-Injektionsspritze nach Anspruch 4, **dadurch gekennzeichnet, dass** das Dichtglied (70) aus einem elastomeren Material hergestellt ist und dass es eine axiale Öffnung (711, 721) aufweist, die sich entlang der Achse (X) erstreckt, um darin die Nadelkanüle (42) passend zu umgreifen.

6. Wegwerf-Injektionsspritze nach Anspruch 4, **dadurch gekennzeichnet, dass** die innere Begrenzungswandoberfläche (33) mit einem deformierbaren Dichtabschnitt (381) ausgebildet ist, der sich in luftdichtem Eingriff mit dem Nabenabschnitt (410) des rohrförmigen Nadelsitzes (41) befindet.

7. Wegwerf-Injektionsspritze nach Anspruch 1 oder 4, ferner **gekennzeichnet durch** ein federndes Element (60), das in dem vorderen umlaufenden Bereich (211) angeordnet ist, um den rohrförmigen Nadelsitz (41) in Richtung zu dem Dichtglied (56) zu federn.

8. Wegwerf-Injektionsspritze nach Anspruch 3, **dadurch gekennzeichnet, dass** der vordere umlaufende Randabschnitt eine umlaufende Nut (39) aufweist, die die Achse (X) umläuft, so dass die innere Begrenzungswandoberfläche (31) in radialen Richtungen bezüglich der Achse (X) stärker verformbar ist.

9. Wegwerf-Injektionsspritze nach Anspruch 3, **dadurch gekennzeichnet, dass** die innere Begrenzungswandoberfläche (31) von dem Greifabschnitt bis zu dem vorderen umlaufenden Randabschnitt (33) abweicht, um einen Reibkontakt zwischen der inneren Begrenzungswandoberfläche (31) und dem Nabenabschnitt (410) des rohrförmigen Nadelsitzes (41) zu verhindern, wenn der rohrförmige Nadelsitz (41) in den Hohlraum (500) eingesaugt wird.

10. Wegwerf-Injektionsspritze nach Anspruch 1, **dadurch gekennzeichnet, dass** der Kolbenkörper (54) einen umlaufenden Dichtring (59) aufweist, der an der oberen Endwand (541) anliegend angeordnet ist, der die Achse (X) umläuft, und der auf und in luftdichtem Eingriff mit dem Abschnitt (22) mit größerem Durchmesser der inneren umlaufenden Zylinderwandoberfläche verschiebbar ist.

11. Wegwerf-Injektionsspritze nach Anspruch 1, **dadurch gekennzeichnet, dass** das Dichtglied (56) eine Eingriffsausnehmung (563) aufweist, die dem Verankerungsabschnitt (412) gegenüber liegt und die sich in Längsrichtung erstreckt, um an dem Verankerungsabschnitt (412) anzugreifen, wenn das Dichtglied (56) von dem Kolbenkörper (54) freigegeben ist, und eine dritte Haltenut (565), die in der Eingriffsausnehmung (563) angeordnet ist und die sich in radialer Richtung relativ zu der Achse (X) erstreckt, wobei der Verankerungsabschnitt (412) einen dritten Haltevorsprung (415) aufweist, der derart angeordnet ist, dass er in der dritten Haltenut (565) gehalten wird, wenn der Verankerungsabschnitt (412) an der Eingriffsausnehmung (563) angreift, derart, dass das Dichtglied (56) zusammen mit dem rohrförmigen Nadelsitz (41) in den Hohlraum (500) hinein gesaugt wird.

12. Wegwerf-Injektionsspritze nach Anspruch 11, **dadurch gekennzeichnet, dass** der Hohlraum (500) einen vorderen und einen hinteren Hohlraumabschnitt (51, 52) umfasst, die benachbart zu der oberen bzw. unteren Endwand (541, 542) angeordnet sind und die einen größeren bzw. kleineren Innendurchmesser aufweisen, wobei das Dichtglied (56) eine äußere umlaufende Dichtoberfläche (564) aufweist, die derart ausgebildet ist, dass sie in verschiebbarem Kontakt und luftdichtem Eingriff mit dem inneren Umfangsrandabschnitt (543) gehalten wird, wenn das Dichtglied (56) in den vorderen Hohlraumabschnitt (51) eingesaugt wird.

13. Wegwerf-Injektionsspritze nach Anspruch 12, **dadurch gekennzeichnet, dass** der Kolben (50) weiter eine Vielzahl an Begrenzungsrippen (58) umfasst, von denen jede benachbart der unteren Endwand (542) angeordnet ist, und von denen jede sich von dem inneren Umfangsrandabschnitt (543) radial und in Richtung zu der Achse (X) erstreckt, um den rohrförmigen Nadelsitz (41) zu fassen, wenn der rohrförmige Nadelsitz (41) zu der unteren Endwand (542) gedrängt wird.

14. Wegwerf-Injektionsspritze nach Anspruch 12, **dadurch gekennzeichnet, dass** der Kolbenkörper (54) ferner eine äußere Umfangswandoberfläche (544) umfasst und eine Vielzahl von Rippenplatten (549), die an der äußeren Umfangswandoberfläche (544) an dem vorderen Hohlraumabschnitt (51) ausgebildet sind, und die bündig mit der äußeren Umfangswandoberfläche (544) an dem hinteren Hohlraumabschnitt (52) sind.

15. Wegwerf-Injektionsspritze nach Anspruch 1, **dadurch gekennzeichnet, dass** die innere umlaufende Zylinderwandoberfläche an der vorderen Öffnung (231) in radialen Richtungen bezüglich der Achse (X) von der Nadelkanüle (42) beabstandet ist, um einen umlaufenden Zwischenraum dazwischen festzulegen.

16. Wegwerf-Injektionsspritze nach Anspruch 15, ferner **gekennzeichnet durch** eine Vielzahl an Rippen (237), die an der inneren umlaufenden Zylinderwandoberfläche angeordnet sind und die winkelmäßig zueinander versetzt angeordnet sind, wobei jede der Rippen (237) sich zu der Nadelkanüle (42) hin erstreckt, wobei ein Luftkanal (236) zwischen zwei benachbarten Rippen (237) ausgebildet ist, um den Durchgang mit der umgebenden Atmosphäre zu verbinden.

17. Wegwerf-Injektionsspritze nach Anspruch 1, ferner **gekennzeichnet durch** zwei injektionsbegrenzende Vorsprünge (545, 25), die an dem Kolbenkörper (54) benachbart zu der unteren Endwand (542) angeordnet sind bzw. an dem Abschnitt (22) mit größerem Durchmesser benachbart zu der hinteren Öffnung (232) und die aneinander anliegen, wenn die obere Endwand (541) den hinteren umlaufenden Randabschnitt (34) erreicht, um das Ausmaß einer Injektionsspeisung zu begrenzen.

18. Wegwerf-Injektionsspritze nach Anspruch 1, **dadurch gekennzeichnet, dass** die untere Endwand (542) eine Öffnung hat, die in Fluidaustausch mit dem Hohlraum (500) steht, wobei der Kolben (50) weiter eine Endabdeckung (57) umfasst, die angeordnet ist, um die Öffnung abzudecken und in luftdichtem Eingriff mit der unteren Endwand (542) zu stehen.

## Revendications

1. Seringue jetable, comprenant :
une canule à aiguille (42),
un siège d'aiguille tubulaire (41) comprenant une partie de raccord (410) qui est disposée pour fixer ladite canule à aiguille (42) à l'intérieur de celui-ci,
un corps cylindrique (20) ayant une surface de paroi de corps cylindrique périphérique interne qui entoure un axe (X) et qui délimite un passage, ledit passage ayant des ouvertures arrière et avant (232, 231) qui sont disposées à l'opposé l'une de l'autre dans une direction longitudinale parallèle à l'axe (X), et
un piston plongeur (50) qui, dans la position d'utilisation, est disposé pour être mobile dans ledit passage, **caractérisée en ce que** ladite surface de paroi de corps cylindrique périphérique interne dudit corps cylindrique (20) comprend un segment de plus grand diamètre (22) et un segment de plus petit diamètre (21) qui délimitent des voies de passage arrière et avant (221, 210) respectivement, et qui sont disposés à proximité desdites ouvertures arrière et avant (232, 231) respectivement, pour former une partie d'épaulement périphérique (222) entre eux, ledit segment de plus petit diamètre (21) comprenant une région périphérique avant (211) et une région périphérique arrière (212) qui est à proximité de ladite partie d'épaulement périphérique (222) et qui a une plus grande dimension que celle de ladite région périphérique avant (211) afin de former une paroi de butée périphérique (213) entre elles, ledit segment de plus grand diamètre (22) comprenant des régions périphériques proximale et distale opposées l'une par rapport à l'autre dans la direction longitudinale et respectivement proximale et distale de ladite partie d'épaulement périphérique (222), ladite région périphérique proximale ayant une zone de retenue qui est espacée de ladite partie d'épaulement périphérique (222) dans la direction longitudinale ;
un élément formant barrière tubulaire (30) qui comprend des parties de bord périphériques avant et arrière (33, 34) opposées l'une par rapport à l'autre dans la direction longitudinale et qui comprend des surfaces de paroi de barrière interne et externe (31, 35) opposées l'une par rapport à l'autre et entourant l'axe (X), ladite surface de paroi de barrière externe (35), dans une position d'utilisation, étant retenue au niveau de ladite zone de retenue du fait d'une première force de frottement créée entre elles tout en étant en prise étanche avec ladite région périphérique proximale, partageant ainsi ladite voie de passage arrière (221) en une chambre compressible (26) qui fait face à ladite partie d'épaulement périphérique (222) et en une chambre de réception (27) qui fait face à ladite partie de bord périphérique arrière (34), ladite surface de paroi de barrière interne (31) ayant un segment de préhension ; ladite partie de raccord (410) dudit siège d'aiguille tubulaire (41) ayant une paroi d'extrémité avant périphérique (411) s'étendant de manière radiale par rapport à l'axe (X), ledit siège d'aiguille tubulaire (41) comprenant en outre :
une partie saisie périphérique qui s'étend à partir de ladite partie de raccord (410) dans la direction longitudinale et à distance de ladite paroi d'extrémité avant périphérique (411), et qui est retenue au niveau dudit segment de préhension du fait d'une seconde force de frottement créée entre ladite partie saisie périphérique et ledit segment de préhension lorsque ladite paroi d'extrémité avant périphérique (411) vient en butée contre ladite paroi de butée périphérique (213) et lorsque ladite canule à aiguille (42) est disposée à l'extérieur de ladite ouverture avant (231), et
une partie d'ancrage (412) s'étendant à partir de ladite partie saisie périphérique dans la direction longitudinale et à distance de ladite partie de raccord (410) ;
ledit piston plongeur (50) comprenant :
un corps (54) de piston plongeur qui comprend une paroi d'extrémité supérieure (541) disposée de manière mobile pour venir en prise contre ladite partie de bord périphérique arrière (34) dudit élément formant barrière tubulaire (30), et une paroi d'extrémité inférieure (542) disposée à l'opposé de ladite paroi d'extrémité supérieure (541) dans la direction longitudinale et s'étendant vers l'extérieur de ladite ouverture arrière (232) afin de pouvoir être actionnée manuellement, ladite paroi d'extrémité supérieure (541) ayant une partie de bord périphérique interne (543) qui entoure l'axe (X) et qui définit une cavité (500) à l'intérieur de celle-ci, ladite cavité (500) s'étendant le long de l'axe (X) et vers ladite paroi d'extrémité inférieure (542) pour contenir un fluide à une pression réduite, et
un élément formant joint d'étanchéité (56) disposé pour être fixé de manière étanche à ladite partie de bord périphérique interne (543) le long d'une ligne d'étanchéité (566) afin de bloquer ledit fluide dans ladite cavité (500), ladite ligne d'étanchéité (566) étant configurée de telle sorte que lorsqu'une force de poussée supérieure aux première et seconde forces de frottement, est appliquée manuellement sur ladite paroi d'extrémité inférieure (542) pour déplacer la paroi d'extrémité supérieure (541) afin de venir en prise contre ladite paroi de bord périphérique arrière (34) dudit élément de barrière tubulaire (30), ledit élément de barrière tubulaire (30) est poussé pour se déplacer vers ladite partie d'épaulement périphérique (222) afin de forcer ledit segment de préhension à se dégager de ladite partie saisie périphérique, de sorte que ladite partie d'ancrage (412), qui reste fixement en place en raison de la butée de ladite paroi d'extrémité avant périphérique (411) contre ladite paroi de butée périphérique (213), est exposée et est impactée par une force d'impact provenant dudit élément formant joint d'étanchéité (56) le long de l'axe (X) de sorte que ladite ligne d'étanchéité (566) est rompue, libérant ainsi ledit élément formant joint d'étanchéité (56) dudit corps (54) de piston plongeur, et permettant ainsi audit siège d'aiguille tubulaire (41) d'être forcé à venir en prise avec ledit élément formant joint d'étanchéité (56) pour être ensuite aspiré dans ladite cavité (500) en raison d'une différence de pression entre l'atmosphère ambiante et la pression réduite.

2. Seringue jetable selon la revendication 1, **caractérisée en ce que** ledit corps cylindrique (20) a une surface de paroi de corps cylindrique périphérique externe (23) qui a une partie de nervure (235) s'étendant dans la direction longitudinale et disposée au niveau de ladite région périphérique avant (211) adjacente à ladite ouverture avant (231), ladite seringue jetable comprenant en outre un protecteur de pointe (43) qui a une extrémité de manchon (433) disposée sur le manchon sur ladite surface de paroi de corps cylindrique périphérique externe (23), ladite extrémité de manchon (433) comprenant une partie de rainure qui se couple avec ladite partie de nervure (235) pour provoquer une mise en prise à cannelure entre ledit protecteur de pointe (43) et ladite surface de paroi de corps cylindrique périphérique externe (23), garantissant ainsi une protection sûre de ladite canule à aiguille (42).

3. Seringue jetable selon la revendication 1, **caractérisée en ce que** l'une parmi ladite région périphérique proximale dans ladite zone de retenue et ladite surface de paroi de barrière externe (35) est formée avec une première rainure de retenue (350), et l'autre parmi ladite région périphérique proximale dans ladite zone de retenue et ladite surface de paroi de barrière externe (35) est formée avec une première saillie de retenue (24) qui vient en prise avec retenue avec ladite première rainure de retenue (350) pour générer la première force de frottement lorsque ladite surface de paroi de barrière externe (35) est dans la position d'utilisation ; l'un parmi ledit segment de préhension de ladite surface de paroi de barrière interne (31) et ladite partie saisie périphérique dudit siège d'aiguille tubulaire (41) est une deuxième rainure de retenue (320), et l'autre parmi ledit segment de préhension de ladite surface de paroi de barrière interne (31) et ladite partie saisie périphérique dudit siège d'aiguille tubulaire (41) est une deuxième saillie de retenue (414) qui vient en prise avec retenue avec ladite deuxième rainure de retenue (320) pour générer la seconde force de frottement lorsque ladite paroi d'extrémité avant périphérique (411) vient en butée contre ladite paroi de butée périphérique (213).

4. Seringue jetable selon la revendication 3, **caractérisée en ce que** ladite paroi d'extrémité avant périphérique (411) comprend un élément d'étanchéité (70) qui est en mise en prise étanche à l'air avec ladite région périphérique arrière (212) afin de maintenir l'étanchéité à l'air de ladite chambre compressible (26), ladite chambre compressible (26) étant remplie avec un fluide, ladite partie de raccord (410) dudit siège d'aiguille tubulaire (41) ayant une pluralité de trous de passage (416) qui est distale de ladite paroi d'extrémité avant périphérique (411) dans la direction longitudinale, qui est formée à travers celle-ci pour être en communication de fluide avec ladite chambre compressible (26) et qui s'étend dans la direction longitudinale de sorte que lorsque ledit élément formant barrière tubulaire (30) est déplacé vers ladite partie d'épaulement périphérique (222), ledit fluide est obligé de s'écouler dans lesdits trous de passage (416), obligeant ainsi ladite partie d'ancrage (412) à se déplacer vers ladite cavité (500) afin d'augmenter la force d'impact.

5. Seringue jetable selon la revendication 4, **caractérisée en ce que** ledit élément d'étanchéité (70) est réalisé à partir d'un matériau élastomère, et a un trou axial (711, 721) s'étendant le long de l'axe (X) pour mettre en prise convenablement ladite canule à aiguille (42) à l'intérieur de celui-ci.

6. Seringue jetable selon la revendication 4, **caractérisée en ce que** ladite surface de paroi de barrière interne (33) est formée avec une partie d'étanchéité déformable (381) qui est en mise en prise étanche à l'air avec ladite partie de raccord (410) dudit siège d'aiguille tubulaire (41).

7. Seringue jetable selon la revendication 1 ou 4, **caractérisée en outre par** un élément de sollicitation (60) disposé dans ladite région périphérique avant (211) pour solliciter ledit siège d'aiguille tubulaire (41) vers ledit élément formant joint d'étanchéité (56).

8. Seringue jetable selon la revendication 3, **caractérisée en ce que** ladite partie de bord périphérique avant a une rainure périphérique (39) entourant l'axe (X) de sorte que ladite surface de paroi de barrière interne (31) est plus déformable dans les directions radiales par rapport à l'axe (X).

9. Seringue jetable selon la revendication 3, **caractérisée en ce que** ladite surface de paroi de barrière interne (31) diverge dudit segment de préhension vers ladite partie de bord périphérique avant (33) pour empêcher le contact de frottement entre ladite surface de paroi de barrière interne (33) et ladite partie de raccord (410) dudit siège d'aiguille tubulaire (41) lorsque ledit siège d'aiguille tubulaire (41) est aspiré dans ladite cavité (500).

10. Seringue jetable selon la revendication 1, **caractérisée en ce que** ledit corps (54) de piston plongeur a une bague d'étanchéité périphérique (59) qui est disposée de manière adjacente à ladite paroi d'extrémité supérieure (541), qui entoure l'axe (X) et qui peut coulisser sur et en mise en prise étanche à l'air avec ledit segment de plus grand diamètre (22) de ladite surface de paroi de corps cylindrique périphérique interne.

11. Seringue jetable selon la revendication 1, **caractérisée en ce que** ledit élément formant joint d'étanchéité (56) a un évidement de mise en prise (563) qui fait face à ladite partie d'ancrage (412) et qui s'étend dans la direction longitudinale afin de mettre en prise ladite partie d'ancrage (412) lorsque ledit élément formant joint d'étanchéité (56) est libéré dudit corps (54) de piston plongeur, et une troisième rainure de retenue (565) qui est disposée dans ledit évidement de mise en prise (563) et qui s'étend dans une direction radiale par rapport à l'axe (X), ladite partie d'ancrage (412) ayant une troisième saillie de retenue (415) qui est disposée pour être retenue dans ladite troisième rainure de retenue (565) lorsque ladite partie d'ancrage (412) vient en prise avec ledit évidement de mise en prise (563) de sorte que ledit élément formant joint d'étanchéité (56), conjointement audit siège d'aiguille tubulaire (41), est aspiré dans ladite cavité (500).

12. Seringue jetable selon la revendication 11, **caractérisée en ce que** ladite cavité (500) comprend des segments de cavité avant et arrière (51, 52) qui sont disposés à proximité desdites parois d'extrémité supérieure et inférieure (541, 542) respectivement, et qui ont de plus petits et de plus grands diamètres internes, respectivement, ledit élément formant joint d'étanchéité (56) ayant une surface de joint d'étanchéité périphérique externe (564) qui est configurée pour être maintenue en contact coulissant et en mise en prise étanche à l'air avec ladite partie de bord périphérique interne (543) lorsque ledit élément formant joint d'étanchéité (56) est aspiré dans ledit segment de cavité avant (51).

13. Seringue jetable selon la revendication 12, **caractérisée en ce que** ledit piston plongeur (50) comprend en outre une pluralité de nervures formant barrière (58), dont chacune est disposée de manière adjacente à ladite paroi d'extrémité inférieure (542) et qui s'étend à partir de ladite partie de bord périphérique interne (543) radialement et vers l'axe (X) pour bloquer ledit siège d'aiguille tubulaire (41) lorsque ledit siège d'aiguille tubulaire (41) est forcé vers ladite paroi d'extrémité inférieure (542).

14. Seringue jetable selon la revendication 12, **caractérisée en ce que** ledit corps (54) de piston plongeur comprend en outre une surface de paroi périphérique externe (544), et une pluralité de plaques de nervures (549) qui est formée sur ladite surface de paroi périphérique externe (544) au niveau dudit segment de cavité avant (51), et qui est de niveau avec ladite surface de paroi périphérique externe (544) au niveau du segment de cavité arrière (52).

15. Seringue jetable selon la revendication 1, **caractérisée en ce que** ladite surface de paroi de corps cylindrique périphérique interne est espacée de ladite canule à aiguille (42) dans les directions radiales par rapport à l'axe (X) au niveau de ladite ouverture avant (231) pour définir un jeu périphérique entre elles.

16. Seringue jetable selon la revendication 15, **caractérisée en outre par** une pluralité de nervures (237) qui est disposée sur ladite surface de paroi de corps cylindrique périphérique interne et qui sont déplacées de manière angulaire l'une par rapport à l'autre, chacune desdites nervures (237) s'étendant vers ladite canule à aiguille (42), un conduit d'air (236) étant formé entre deux nervures adjacentes desdites nervures (237) pour faire communiquer ledit passage avec l'atmosphère ambiante.

17. Seringue jetable selon la revendication 1, **caractérisée en outre par** deux saillies de limite d'injection (545, 25) qui sont disposées sur ledit corps (54) de piston plongeur adjacent à ladite paroi d'extrémité inférieure (542) et ledit segment de plus grand diamètre (22) adjacent à ladite ouverture arrière (232) respectivement, et qui viennent en butée l'une contre l'autre lorsque ladite paroi d'extrémité supérieure (541) atteint ladite partie de bord périphérique arrière (34) afin de limiter une étendue d'une course d'injection.

18. Seringue jetable selon la revendication 1, **caractérisée en ce que** ladite paroi d'extrémité inférieure (542) a une ouverture qui est en communication de fluide avec ladite cavité (500), ledit piston plongeur (50) comprenant en outre un capuchon d'extrémité (57) qui est disposé pour recouvrir ladite ouverture et pour être en mise en prise étanche à l'air avec ladite paroi d'extrémité inférieure (542).
